# EUROPÄISCHE PATENTANMELDUNG

(11) **EP 1 321 100 A2**
(43) Veröffentlichungstag der Anmeldung: **25.06.2003**
(21) Anmeldenummer: 02102807.1
(22) Anmeldetag: 18.12.2002
(51) Int. Cl.: A61B 6/03

(54) **Computertomograph**

(30) Priorität: 20.12.2001 DE 10162768
(71) Anmelder: Philips Intellectual Property & Standards GmbH, 20099 Hamburg (DE); Koninklijke Philips Electronics N.V., 5621 BA Eindhoven (NL)
(72) Erfinder: Proksa, Roland, 52088, Aachen (DE)
(74) Vertreter: Volmer, Georg, Dipl.-Ing.

(57) **Zusammenfassung**

Die Erfindung betrifft einen Computertomographen, bei dem mit einem kegelförmigen Strahlenbündel entlang einer helixförmigen Abtastbahn Messdaten eines Patienten erfasst werden. Dabei ist die Abmessung des Detektorfensters um einen Faktor 3,5,7...größer als der Abstand benachbarter Windungen der Helix. Um aus den erfassen redundanten Daten zur vollständigen Füllung des Radon-Raumes für eine exakte Rekonstruktion die geeigneten Messdaten auszuwählen, wind erfindungsgemäß vorgeschlagen, dass eine Herzbewegungssignaldetektionseinrichtung vorgesehen ist zur Detektion eines die Herzbewegung repräsentierenden Herzbewegungssignals und dass die Rekonstruktionseinheit ausgestaltet ist zur Auswahl derjenigen Messdaten aus den den Radon-Raum bereichsweise redundant füllenden Messdaten anhand des Herzbewegungssignals derart, dass der Radon-Raum mittels Messdaten aus möglichst bewegungsarmen Herzbewegungsphasen vollständig und homogen gefüllt ist.

## Beschreibung

Die Erfindung betrifft einen Computertomographen mit
- einer Abtasteinheit mit einer Strahlenquelle und einer damit verbundenen Detektoreinheit zur Erfassung eines von der Strahlenquelle emittierten, kegelförmigen Strahlenbündels nach Durchtreten durch den Untersuchungsbereich eines zwischen Strahlenquelle und Detektoreinheit befindlichen Patienten,
- einer Antriebseinheit zur Erzeugung einer um eine Rotationsachse verlaufenden, helixförmigen Relativbewegung zwischen der Abtasteinheit und dem Patienten und
- einer Rekonstruktionseinheit zur Rekonstruktion eines 3D-Bilddatensatzes des Untersuchungsberichts aus den von der Detektoreinheit innerhalb eines durch die Helix definierten Detektorfensters erfassten Messdaten, wobei die Verbindungsgeraden der Strahlenquelle mit den beiden in Richtung der Rotationsachse gegeneinander versetzten Rändern des Detektorfensters zwei in Richtung der Rotationsachse um die Strecke (2n+1)p versetzte Abschnitte der Helix schneiden, wobei n eine kleine ganze Zahl größer oder gleich 1 ist und p dem axialen Versatz zweier benachbarter Windungen der Helix entspricht, so dass der Radon-Raum mittels der Messdaten bereichsweise mit unterschiedlich großer Redundanz gefüllt wird.

Ein solcher Computertomograph (im folgenden auch CT-Gerät genannt) ist aus der EP 0 981 995 A2 bekannt. Bei diesem Computertomographen weist die Abtastbahn die Form einer Helix auf, und ein kegelförmiges Strahlenbündel durchsetzt den Untersuchungsbereich eines Untersuchungsobjekts, z.B. eines Patienten. Weiter wird dort vorgeschlagen, die Abmessungen des Detektorfensters (oder des für die Rekonstruktion herangezogenen Teils davon) um einen Faktor 3,5,7...größer zu wählen als den Abstand benachbarter Windungen der Helix. Bei einer solchen Geometrie wird dann jedes Voxel im Untersuchungsbereich exakt aus einem Winkelbereich von 3π, 5π, 7π... bestrahlt, wenn es den Strahlenkegel passiert. Dadurch wird der Radon-Raum zumindest bereichsweise mit mehrfacher z.B. 3,5,7 facher, Redundanz gefüllt. Durch eine solche Datenakquisition lässt sich letztlich eine verbesserte Bildqualität erreichen.

Der Einsatz der Computertomographie zur Bildgebung im Herzbereich führt häufig zu Bildern mit Artefakten aufgrund der Herzbewegung während der Datenerfassung. Um diese Artefakte zu reduzieren, werden häufig Rekonstruktionsmethoden angewendet, bei denen ein während der Messdatenerfassung zusätzlich erfasstes Herzbewegungssignal, beispielsweise ein EKG-Signal, ausgewertet wird, um nur während Herzbewegungsphasen mit geringer Bewegung erfasste Messdaten zur Rekonstruktion zu verwenden. Allerdings muss dabei sichergestellt werden, dass zur Rekonstruktion ausreichend Daten aus solchen Herzbewegungsphasen mit geringer Bewegung des Herzen vorliegen, um überhaupt eine Rekonstruktion eines 3D-Bilddatensatzes durchführen zu können.

Ein weiteres Problem besteht darin, dass der interessierende Untersuchungsbereich eines Patienten größer ist als das Volumen, dass mit einer kreisförmigen Trajektorie gescannt werden kann. Es werden deshalb häufig helixförmige Trajektorien zur Messdatenerfassung verwendet, wobei dann Messdaten redundant erfasst werden müssen, um die beschriebene Rekonstruktion mit Auswertung des Herzbewegungssignals (sogenannte "gated reconstruction") durchführen zu können.

Der Erfindung liegt die Aufgabe zugrunde, einen Computertomographen der eingangs genannten Art weiter zu verbessern und insbesondere eine exakte Rekonstruktion zu ermöglichen.

Diese Aufgabe wird erfindungsgemäß dadurch gelöst, dass eine Herzbewegungssignaldetektionseinrichtung vorgesehen ist zur Detektion eines die Herzbewegung repräsentierenden Herzbewegungssignals und dass die Rekonstruktionseinheit ausgestaltet ist zur Auswahl derjenigen Messdaten aus den den Radon-Raum bereichsweise redundant füllenden Messdaten anhand des Herzbewegungssignals derart, dass der Radon-Raum mittels Messdaten aus möglichst bewegungsarmen Herzbewegungsphasen vollständig und homogen gefüllt ist.

Der Erfindung liegt dabei die Erkenntnis dabei zugrunde, dass anhand des während der Messdatenerfassung detektierten Herzbewegungssignals aus den redundanten Daten geeignete Daten ausgewählt werden können, so dass der Radon-Raum homogen und vollständig sowie möglichst nicht-redundant gefüllt ist, um eine exakte Rekonstruktion eines 3D-Datensatzes zu ermöglichen. Geeigneterweise werden solche Messdaten ausgewählt, die aus Herzbewegungsphasen mit möglichst geringer Bewegung, beispielsweise bei einem EKG kurz vor Auftreten der R-Zacke, erfasst wurden. Die Rekonstruktion kann dann mittels des beispielsweise in "The n-PI-Method for Helical Cone-Beam CT", R. Proksa, Th. Köhler, M. Grass, J. Timmer, IEEE Transactions and Medical Imaging, vol. 19, no.9,2000 beschriebenen Rekonstruktionsverfahren erfolgen.

Vorteilhafte Ausgestaltungen des erfindungsgemäßen Computertomographen sind in den Unteransprüchen angegeben. Gemäß der bevorzugten Ausgestaltung nach Anspruch 2, wonach der Parameter n vorzugsweise den Wert 1 oder 2 aufweist, beträgt der Abstand zwischen den Rändern des Detektorfensters das Dreifache bzw. Fünffache des Abstandes zweier Windungen der Helix. Das Detektorfenster kann wie bei den bekannten Computertomographen dadurch realisiert werden, dass die Detektoreinheit und/oder das von der Strahlenquelle durch einen Kollimator ausgeblendete kegelförmige Strahlenbündel entsprechend geformt werden.

Als Herzbewegungssignaldetektionseinrichtung wird bevorzugt ein Elektrokardiograph zur Messung eines Elektrokardiogramms während der Messdatenerfassung eingesetzt. Alternativ kann das Herzbewegungssignal auch direkt aus den erfassten Messdaten ermittelt werden. So ist beispielsweise bekannt, dass der Schwerpunkt einer erfassten Messdatenverteilung bzw. eines Bildes in den rekonstruierten Projektionsdaten erhalten bleibt. Der Schwerpunkt verändert sich jedoch im allgemeinen in seiner örtlichen Lage mit der Bewegung der Anatomie, beispielsweise mit der Herzbewegung. Dies kann vorteilhaft ausgenutzt werden, um aus Veränderungen der Lage des Schwerpunktes verschiedener Projektionsdaten bzw. Bilder ein Herzbewegungssignal abzuleiten.

Bei dem erfindungsgsmäßen Computertomographen werden nicht alle Bereiche des Radon-Raums mit gleich großer Redundanz gefüllt. In jedem Bereich liegen alle Radon-Ebenen, die eine bereichsbezogene Anzahl von Überschneidungen mit der helixförmigen Trajektorie der Strahlenquelle aufweisen. In anderen Worten, in einem Bereich liegen alle Radon-Ebenen mit einer einzigen Überschneidung mit der Helix, in einem anderen Bereich liegen alle Radon-Ebenen mit drei Überschneidungen mit der Helix usw. Es lassen sich somit unterschiedliche Kategorien von Bereichen unterscheiden. In einer bevorzugten Ausgestaltung ist deshalb vorgesehen, dass in Bereichen mit einfacher oder mehrfacher Redundanz nach Auswahl hinsichtlich der Herzbewegungsarmut geeigneter Messdaten eine Mittelung der geeigneten Messdaten erfolgt, um diesen Bereich des Radon-Raums mit reduzierter Redundanz zu füllen. Sofern andererseits die Auswahl hinsichtlich der Bewegungsarmut ergibt, dass bereichsweise gar keine Messdaten verwendet werden können zur Füllung des Radon-Raumes in diesem Bereich, kann auch eine Interpolation aus benachbarten Messdaten erfolgen.

Gemäß einer weiteren Ausgestaltung der Erfindung ist vorgesehen, dass die Auswahl der zur Füllung des Radon-Raums verwendeten Messdaten derart erfolgt, dass die redundanten Bereiche des Radon-Raums in Unterbereiche, vorzugsweise in n Unterbereiche, unterteilt werden und dass anhand des Herzbewegungssignals Unterbereiche ausgewählt werden, deren zugeordnete Messdaten den Radon-Raum aus möglichst bewegungsarmen Herzbewegungsphasen vollständig und homogen füllen. Bevorzugt ist der Radon-Raum in dreieckförmige Bereiche unterteilt, die wiederum in individuelle Bereiche unterteilt sind. Jeder dieser Unterbereiche ist Segmenten des Detektorfensters zugeordnet, die durch die helixförmige Trajektorie gebildet sind. Die dreieckförmigen Bereiche werden somit wiederum in kleinere Dreiecke, bevorzugt n Dreiecke unterteilt. Da sich in redundanten Bereichen des Radon-Raums Unterbereiche überlappen, wodurch sich die genannte Redundanz ergibt, wird gemäss dieser Ausführungsform anhand des Herzbewegungssignals die Auswahl der Unterbereiche derart vorgenommen, dass der Radon-Raum letztendlich wieder vollständig und homogen gefüllt ist. Bei dreieckförmigen Unterbereichen wird der Radon-Bereich also aus geeigneten dreieckförmigen Unterbereichen vollständig ausgefüllt.

In einer vorteilhaften Ausgestaltung kann weiter vorgesehen sein, dass bei der Mittelung mehrerer Messdaten eine Gewichtung der Messdaten erfolgt, wobei die Gewichtung abhängig von der Stärke der bei der Erfassung der einzelnen Messdaten erfassten Herzbewegung ist. Dies bedeutet also, dass Messdaten, die bei geringerer Herzbewegung erfasst werden, bei einer Mittelung stärker gewichtet werden als Messdaten, die aus einer Herzbewegungsphase mit stärkerer Herzbewegung stammen. Auch dadurch werden letztlich eine verbesserte Rekonstruktion und eine erhöhte Bildqualität erreicht.

Die Erfindung betrifft auch ein Computertomographie Verfahren, wie es in Anspruch 8 angegeben ist. Dieses Verfahren kann in ähnlicher oder identischer Weise weiter gebildet sein wie der beschriebene erfindungsgemäße Computertomograph.

Die Erfindung wird nachfolgend anhand der Zeichnungen näher erläutert. Es zeigen:
- Figur 1: einen erfindungsgemäßen Computertomographen in schematischer Darstellung,
- Figur 2: eine Ausgestaltung einer helixförmigen Abtastbahn,
- Figur 3: die Abwicklung einer Detektoreinheit,
- Figur 4: eine Radon-Ebene des Radon-Raums mit einer Anzahl von Strahlenquellenpositionen, und
- Figur 5: eine Radon-Ebene mit verschiedenen Unterbereichen.

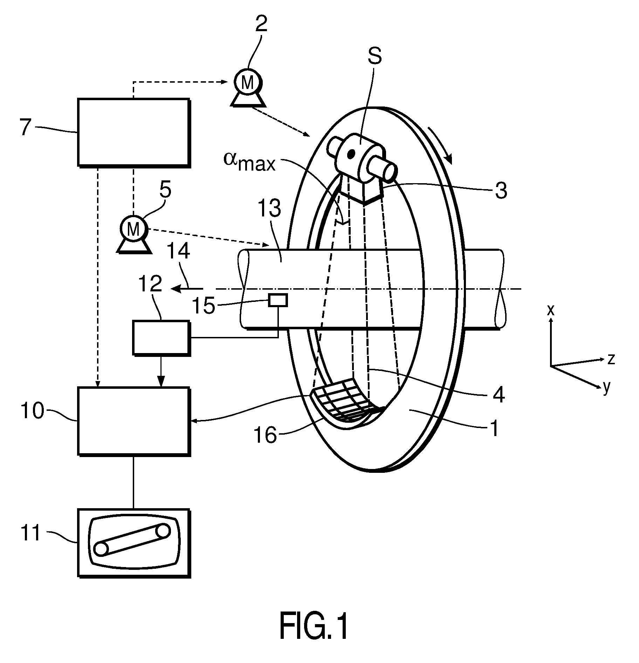

Der in Figur 1 dargestellte Computertomograph umfasst eine Gantry 1, die um eine parallel zur z-Richtung verlaufende Rotationsachse 14 rotieren kann. Dazu wird die Gantry 1 von einem Motor 2 mit einer vorzugsweise konstanten, aber einstellbaren Winkelgeschwindigkeit angetrieben. An der Gantry 1 ist eine Strahlenquelle S, beispielsweise eine Röntgenröhre, befestigt. Diese ist mit einer Kollimatoranordnung 3 versehen, die aus der von der Strahlenquelle S erzeugten Strahlung ein kegelförmiges Strahlenbündel 4 ausblendet. Das Strahlenbündel 4 durchdringt einen nicht näher dargestellten Patienten, der sich in einem zylinderförmigen Untersuchungsbereich 13 befindet. Nach dem Durchsetzen des Untersuchungsbereichs 13 trifft das Röntgenstrahlenbündel 4 auf eine an der Gantry 1 befestigte zweidimensionale Detektoreinheit 16.

Der mit α ₘₐₓ bezeichnete Öffnungswinkel des Strahlenbündels 4 (als Öffnungswinkel ist der Winkel definiert, den ein in der x-y-Ebene am Rande liegender Strahl des Bündels 4 mit einer durch die Strahlenquelle S und die Rotationsachse 14 definierten Ebene einschließt) bestimmt dabei den Durchmesser des Untersuchungsbereichs 13, innerhalb dessen das zu untersuchende Objekt sich bei der Akquisition der Messwerte befinden muss. Der dabei beispielsweise auf einem Patientenlagerungstisch liegende, im Untersuchungsbereich 13 angeordnete Patient kann mittels eines Motors 5 parallel zur Richtung der Rotationsachse 14 bzw. der z-Achse verschoben werden.

Die von der Detektoreinheit 16 akquirierten Messdaten werden einer Rekonstruktionseinheit 10 zugeführt, die daraus die Absorptionsverteilung in dem vom Strahlenkegel 4 erfassten Teil des Untersuchungsbereichs 13 rekonstruiert und beispielsweise auf einem Monitor 11 wiedergibt. Die beiden Motoren 2 und 5, die Rekonstruktionseinheit 10, die Strahlenquelle S und der Transfer der Messdaten von der Detektoreinheit 16 zur Rekonstruktionseinheit 10 werden von einer geeigneten Steuereinheit 7 gesteuert.

Die Steuerung der Motoren 2 und 5 kann derart erfolgen, dass das Verhältnis der Vorschubgeschwindigkeit des Untersuchungsbereichs 13 und die Winkelgeschwindigkeit der Gantry 1 in einem konstanten Verhältnis stehen, so dass sich Strahlenquelle S und Untersuchungsbereich 13 relativ zueinander auf einer helixförmigen Bahn, der sogenannten Trajektorie, bewegen. Dabei ist es gleichgültig, ob die Abtasteinheit aus Strahlenquelle S und Detektoreinheit 16 oder der Untersuchungsbereich 13 die Rotations- bzw. Vorschubbewegung ausführen; wesentlich ist allein die Relativbewegung.

Erfindungsgemäß wird gleichzeitig bei der gezeigten Ausgestaltung der Erfindung zur Erfassung der Messdaten ein Herzbewegungssignal mittels eines Elektrokardiographen 12 und eines an einen Patienten angebrachten Sensors 15 erfasst. Dieses Signal wird ebenfalls der Rekonstruktionseinheit 10 zugeführt, um dadurch die Auswahl der für die Rekonstruktion geeigneten Messdaten so vorzunehmen, dass möglichst nur aus bewegungsarmen Herzbewegungsphasen erfasste Messdaten verwendet werden. Es sei an dieser Stelle nochmals erwähnt, dass das Herzbewegungssignal auch auf andere Weise als mittels eines EKG gewonnen werden kann, beispielsweise direkt oder indirekt aus den von der Abtasteinheit erfassten Messdaten.

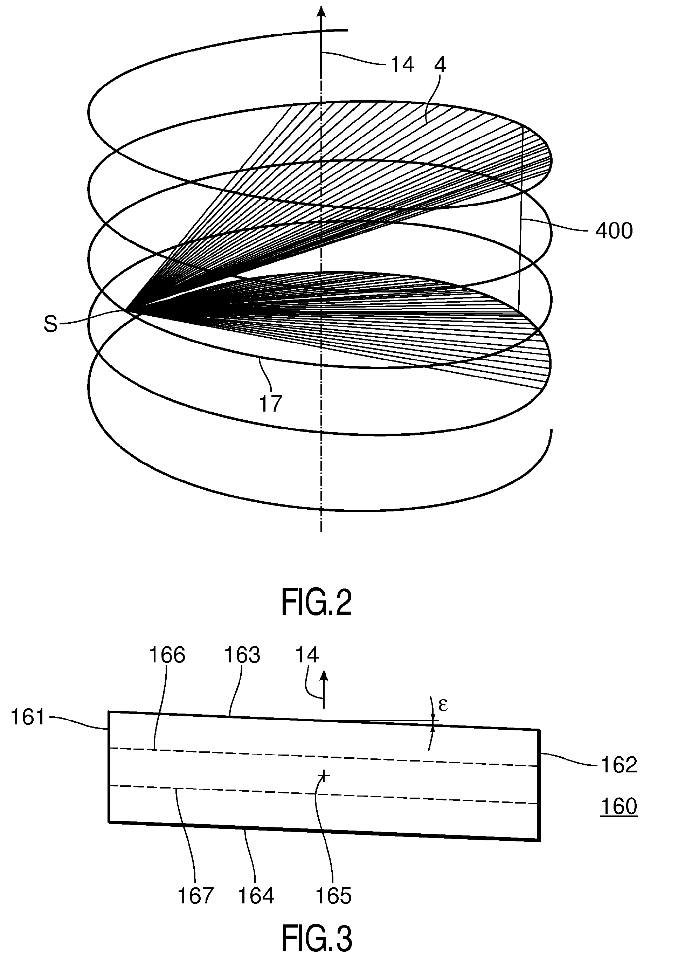

In Figur 2 ist eine helixförmige Trajektorie 17, auf der sich die Strahlenquelle S und die (nicht gezeigte) Detektoreinheit 16 um den (nicht gezeigten) Untersuchungsbereich 13 bewegen. Das von der Strahlenquelle S emittierte kegelförmige Strahlenbündel kann man sich aus einer Vielzahl von Strahlenfächern denken, die sich in zur Rotationsachse 14 parallelen Ebenen befinden, die sich in der Strahlenquelle S schneiden. Obwohl dieses Strahlenbündel auch in anderen Ebenen fächerförmig gruppierte Strahlen enthält, sollen im Folgenden nur solche Strahlenkombinationen als Strahlenfächer bezeichnet werden, die - wie der Strahlenfächer 400 - in zur Rotationsachse 14 parallelen Ebenen liegen. Der Öffnungswinkel α ₘₐₓ des Strahlenbündels 4 ist so bemessen, dass er mit seinen äußeren Fächern den Untersuchungsbereich gerade tangiert. Wenn αₘₐₓ = 30° ist, ist der Radius des Untersuchungsbereichs 13 gerade halb so gut wie der Radius der helixförmigen Bahn. Die Öffnung der Kollimatoranordnung 3 ist so gestaltet, dass zwei der Strahlenquelle gegenüberliegende, gegeneinander um die Strecke 3p versetzte (p entspricht dem Vorschub in z-Richtung während einer vollständigen Umdrehung der Strahlenquelle S) Bindungen der Helix 17 mit den Strahlen am oberen und unteren Rand des Strahlenbündels 4 zusammenfallen.

Der obere und untere Rand des Detektorfensters fällt mit der Projektion der Windungen der Helix 17 bzw. deren der Strahlenquelle gegenüberliegenden Abschnitten auf die Detektoreinheit zusammen, d.h. die von der Strahlenquelle ausgehenden Verbindungsgeraden mit den erwähnten Rändern schneiden diese Windungen.

Figur 3 stellt die Abwicklung des Detektorfensters 160 von dem durch die Helix 17 definierten Zylinder in die Zeichenebene dar. Die Abwicklung hat die Form eines Parallelogramms mit zur z-Richtung parallelen Seiten 161,162, deren Abstand voneinander um so größer ist, je größer der Öffnungswinkel α ₘₐₓ des Strahlenbündels ist. Für die Länge dieser Seiten, d.h. die Höhe h des Detektorfensters, gilt h=3p. Der obere und untere Rand 163 bzw. 164 des Detektorfensters 160 schließt mit der Senkrechten auf die Rotationsachse 14 einen Winkel ε ein, der sich nach der Beziehung tan ε =p/2πR berechnen lässt, wobei R der Abstand der Strahlenquelle von der Rotationsachse ist. Dabei ist angenommen, dass die Vorschubgeschwindigkeit und die Rotationsgeschwindigkeit konstant sind. In Figur 3 ist außerdem noch der Mittelpunkt 165 des Detektorfensters 160 eingezeichnet; mit den gestrichelten Linien 166 und 167 ist die Projektion der beiden Windungen bezeichnet, die zwischen den Windungen der Helix 17 liegen, die mit dem oberen Rand 163 bzw. dem unteren Rand 164 zusammenfallen.

Jeder Punkt im Untersuchungsbereich wird bei seinem Eintritt in das kegelförmige Strahlenbündel 4 auf den unteren Rand 164 und bei seinem Austritt aus dem Strahlenbündel 4 auf den oberen Rand 163 projiziert. Es lässt sich zeigen, dass die Strahlenquelle S um den betreffenden Punkt eine Drehung von genau 3π vollführt, während seine Projektion sich vom unteren Rand 164 zum oberen Rand 163 bewegt. Bezogen auf die Rotationssachse 14 kann die von der Strahlenquelle S ausgeführte Drehbewegung aber größer oder kleiner als 3π sein. Hinsichtlich weiterer Einzelheiten dieses n-PI-Verfahrens sei auf die eingangs bereits genannte EP 0 981 995A2 verwiesen, deren Beschreibung als in die vorliegende Anmeldung aufgenommen gelten soll.

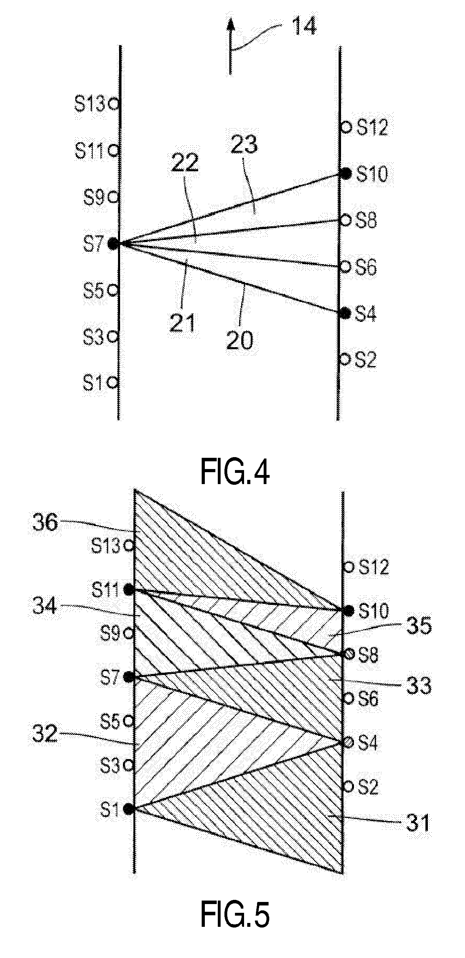

Mit dem beschriebenen Computertomographen lässt sich das n-PI-Verfahren durchführen, bei dem einzelne Bereiche des Radon-Raums mittels der akquirierten Messdaten mit unterschiedlich großer Redundanz - je nach Wahl von n gefüllt werden. In dem gezeigten Beispiel beträgt n=3, so dass einzelne Bereiche eine dreifache Redundanz aufweisen. Bei den n-PI-Verfahren werden die Radon-Ebenen des Radon-Raumes in dreieckförmige Bereiche unterteilt. Eine einzelne Radon-Ebene mit einem solchen dreieckförmigen Bereich 20 ist in Figur 4 gezeigt. Darin ist eine seitliche Ansicht der helixförmigen Trajektorie im Querschnitt gezeigt, so dass die Schnittpunkte der Radon-Ebene mit der Trajektorie an Positionen S1 bis S13 für einen 3π-Scan gezeigt sind. Als Beispiel ist der dreieckförmige Bereich 20 gezeigt, der von der Strahlenquelle an der Position S7 beleuchtet wird. Wie in dem oben genannten Artikel von R. Proksa u.a. gezeigt ist, bedeckt die Kombination aller dreieckförmiger Bereiche, die von den einzelnen Strahlenquellenpositionen beleuchtet werden, die gezeigte Radon-Ebene genau dreimal.

Im allgemeinen erfordert das n-PI-Verfahren die Berechnung der ersten radialen Ableitung des Radon-Raums. Diese Werte können aus divergierenden 2D Fächerstrahlprojektionssegmenten berechnet werden, die die Radon-Ebene schneiden. Im folgenden wird erläutert, wie quasi-vollständige und homogene, sowie möglichst nicht-redundante Bereiche aller Radon-Ebenen erreicht werden. Abhängig von dem Grad der Redundanz kann es jedoch möglich sein, dass nicht alle erforderlichen Radon-Werte aus den akquirierten Messdaten berechnet werden können. Es wird jedoch angenommen, dass der Radon-Raum mit ausreichender Dichte gefüllt ist, um eine einfache Interpolation fehlender Radon-Werte zu ermöglichen.

Dabei ist zunächst die Akquisition von Messdaten mit der n-PI-Methode wie oben beschrieben vorausgesetzt. Der Radon-Raum wird in Bereiche unterteilt. In jedem Bereich liegen alle Radon-Ebenen vor, die eine bereichsbezogene Anzahl von Schnittpunkten mit der helixförmigen Trajektorie der Strahlenquelle aufweisen. In anderen Worten, alle Radon-Ebenen mit einem Schnittpunkt mit der Helix liegen in einem ersten Bereich, alle Radon-Ebenen mit 3 Schnittpunkten liegen in einem zweiten Bereich usw. Die Anzahl der Schnittpunkte sei mit m bezeichnet. Es können dann drei unterschiedliche Kategorien von Bereichen unterschieden werden.
a) Der Bereich mit m=1: Diese Radon-Ebenen sind nur ein einziges Mal beleuchtet. Wenn das Herzbewegungssignal die Benutzung der ermittelten Daten ermöglicht, d.h., wenn die Erfassung dieser Messdaten in einer Herzbewegungsphase mit ausreichend geringer Herzbewegung erfolgte, werden diese Messdaten benutzt. Anderenfalls müssen die fehlenden Radon-Werte von Nachbarwerten interpoliert werden.
b) Die Bereiche mit 1<m≤n: Diese Radon-Ebene ist vollständig beleuchtet durch alle m Strahlenquellenpositionen. Es wird zunächst festgestellt, welche Messdaten von welchen Strahlenquellenpositionen entsprechend dem Herzbewegungssignal benutzt werden können. Wenn keine Strahlenquellen benutzt werden können, werden die fehlenden Radon-Werte interpoliert aus Nachbarwerten. Wenn Messdaten von einer oder mehreren Strahlenquellenpositionen benutzt werden können, wird Vorteilhafterweise eine Mittelung der Messdaten vorgenommen.
c) Die Bereiche mit m>n: In diesen Bereichen wird zunächst eine feinere Segmentierung der Messdaten vorgenommen. Jeder dreieckförmige Bereich wird weiter unterteilt in n individuelle Unterbereiche. Solche Unterbereiche 21,22,23 des dreieckförmigen Bereichs 20 sind auch in Figur 4 gezeigt. Jedem dieser Unterbereiche sind Segmente des Detektors zugeordnet, die durch die Unterteilung des Detektors mittels der helixförmigen Trajektorie entstehen. In anderen Worten, der Detektor wird in ein inneres PI-Fenster, den oberen und unteren Teil bzgl. des 3-PI-Fensters usw. unterteilt, bis letztendlich n Unterbereiche entstehen. Auf der Basis dieser Unterteilung erfolgt die Auswahl der geeigneten Messdaten und die Suche nach möglichen Kombinationen der Unterbereiche so, dass die Radon-Ebene vollständig und homogen ausgefüllt ist. Wenn auch hier wiederum keine möglichen Kombinationen gefunden werden, werden fehlende Radon-Werte interpoliert. Bei mehrfach verwendbaren Messdaten kann wiederum eine Mittelung vorgenommen werden.

In Figur 5 ist eine Radon-Ebene gezeigt, die vollständig durch eine geeignete Kombination von dreieckförmigen Unterbereichen 31-36 gezeigt. Die Daten für die Füllung dieser Unterbereiche 31-36 entstammen dabei Messdaten, die an Strahlenquellenpositionen S1, S4, S7, S8, S10 und S11 akquiriert wurden. Wie leicht zu erkennen ist, werden dabei nicht an jeder Strahlenquellenposition alle erfassten Messdaten verwendet, sondern beispielsweise werden von den an der Strahlenquellenposition S11 erfassten Messdaten nur die im Teilbereich 35 liegenden Messdaten ausgewertet zur Füllung der Radon-Ebene.

Das Verfahren zur Auswahl der Messdaten kann so modifiziert werden, dass die verwendbaren Strahlenquellenpositionen bzw. Messdaten unterschiedliche Prioritäten erhalten basierend auf einer Schätzung, wie nahe sie an der Herzphase liegen, d. h wie stark die Herzbewegung bei der Erfassung der entsprechenden Messdaten war. Je stärker die Herzbewegung war, um so geringer können die Messdaten gewichtet werden, sofern eine Kombination der Messdaten zur Füllung der Radon-Ebene vorgenommen wird.

Mit dem erfindungsgemäßen Computertomographen und Verfahren lässt sich eine vollständige Füllung des Radon-Raums vornehmen, um eine exakte Rekonstruktion eines 3D-Datensatzes zu erzielen. Dabei werden erfindungsgemäß Messdaten aus möglichst bewegungsarmen Herzbewegungsphasen ausgewählt, um eine möglichst hohe Bildqualität zu erreichen. Die Rekonstruktion selbst kann dabei mit bekannten Verfahren erfolgen. Ein solches Rekonstruktionsverfahren ist sowohl in der EP 0 981 995 A2 als auch in dem erwähnten Artikel von R. Proksa u.a. beschrieben, worauf hiermit explizit Bezug genommen wird.

## Patentansprüche

1. Computertomograph mit
- einer Abtasteinheit mit einer Strahlenquelle (S) und einer damit verbundenen Detektoreinheit (16) zur Erfassung eines von der Strahlenquelle (S) emittierten, kegelförmigen Strahlenbündels nach Durchtreten durch den Untersuchungsbereich (13) eines zwischen Strahlenquelle und Detektoreinheit befindlichen Patienten,
- einer Antriebseinheit (2,5) zur Erzeugung einer um eine Rotationsachse (14) verlaufenden, helixförmigen Relativbewegung (17) zwischen der Abtasteinheit und dem Patienten, und
- einer Rekonstruktionseinheit (10) zur Rekonstruktion eines 3D-Bilddatensatzes des Untersuchungsbereichs (13) aus den von der Detektoreinheit (16) innerhalb eines durch die Helix (17) definierten Detektorfensters erfassten Messdaten, wobei die Verbindungsgeraden der Strahlenquelle mit den beiden in Richtung der Rotationsachse gegeneinander versetzten Rändern (163,164) des Detektorfensters (160) zwei in Richtung der Rotationsachse (14) um die Strecke (2n+1)p versetzte Abschnitte der Helix (17) schneiden, wobei n eine kleine ganze Zahl größer oder gleich 1 ist und p dem achsialen Versatz zweier benachbarter Windungen der Helix (17) entspricht, so dass der Radon-Raum mittels der Messdaten bereichsweise mit unterschiedlich großer Redundanz gefüllt wird,
**dadurch gekennzeichnet,**
**dass** eine Herzbewegungssignaldetektionseinrichtung (12,15) vorgesehen ist zur Detektion eines die Herzbewegung repräsentierenden Herzbewegungssignals und dass die Rekonstruktionseinheit (10) ausgestaltet ist zur Auswahl derjenigen Messdaten aus den den Radon-Raum bereichsweise redundant füllenden Messdaten anhand des Herzbewegungssignals derart, dass der Radon-Raum mittels Messdaten aus möglichst bewegungsarmen Herzbewegungsphasen vollständig und homogen gefüllt ist.

2. Computertomograph nach Anspruch 1,
**dadurch gekennzeichnet,**
**dass** n gleich 1 oder 2 ist.

3. Computertomograph nach Anspruch 1,
**dadurch gekennzeichnet,**
**dass** die Herzbewegungssignaldetektionseinrichtung (12,15) ein Elektrokardiograph ist.

4. Computertomograph nach Anspruch 1,
**dadurch gekennzeichnet,**
**dass** die Herzbewegungssignaldetektionseinrichtung Mittel aufweist zur Ableitung des Herzbewegungssignals aus den erfassten Messdaten.

5. Computertomograph nach Anspruch 1,
**dadurch gekennzeichnet,**
**dass** die Auswahl der zur Füllung des Radon-Raums verwendeten Messdaten derart erfolgt, dass bei mehreren verwendbaren Messdaten eine Mittelung erfolgt und dass bei keine verwendbaren Messdaten eine Interpolation aus benachbarten Messdaten erfolgt.

6. Computertomograph nach Anspruch 1,
**dadurch gekennzeichnet,**
**dass** die Auswahl der zur Füllung des Radon-Raums verwendeten Messdaten derart erfolgt, dass die redundanten Bereiche des Radon-Raums in Unterbereiche, vorzugsweise in n Unterbereiche, unterteilt werden und dass anhand des Herzbewegungssignals Unterbereiche ausgewählt werden, deren zugeordnete Messdaten den Radon-Raum aus möglichst bewegungsarmen Herzbewegungsphasen vollständig und homogen füllen.

7. Computertomograph nach Anspruch 1,
**dadurch gekennzeichnet,**
**dass** bei der Mittelung mehrerer Messdaten eine Gewichtung der Messdaten erfolgt, wobei die Gewichtung abhängig von der Stärke der bei der Erfassung der einzelnen Messdaten erfassten Herzbewegung ist.

8. Computertomographie-Verfahren mit den Schritten:
- Erfassung eines von der Strahlenquelle (S) emittierten, kegelförmigen Strahlenbündels nach Durchtreten durch den Untersuchungsbereich (13) eines zwischen Strahlenquelle (S) und Detektoreinheit (16) befindlichen Patienten mittels einer Abtasteinheit mit einer Strahlenquelle (S) und einer damit verbundenen Detektoreinheit (16),
- Erzeugung einer um eine Rotationsachse (14) verlaufenden, helixförmigen Relativbewegung zwischen der Abtasteinheit und dem Patienten mittels einer Antriebseinheit (2,5), und
- Rekonstruktion eines 3D-Bilddatensatzes des Untersuchungsbereichs (13) aus den von der Detektoreinheit (16) innerhalb eines durch die Helix (17) definierten Detektorfensters erfassten Messdaten mittels einer Rekonstruktionseinheit (10), wobei die Verbindungsgeraden der Strahlenquelle (S) mit den beiden in Richtung der Rotationsachse (14) gegeneinander versetzten Rändern des Detektorfensters zwei in Richtung der Rotationsachse (14) um die Strecke (2n+1)p versetzte Abschnitte der Helix (17) schneiden, wobei n eine kleine ganze Zahl größer oder gleich 1 ist und p dem achsialen Versatz zweier benachbarter Windungen der Helix (17) entspricht, so dass der Radon-Raum mittels der Messdaten bereichsweise mit unterschiedlich großer Redundanz gefüllt wird,
**dadurch gekennzeichnet,**
**dass** ein die Herzbewegung repräsentierenden Herzbewegungssignals mittels einer Herzbewegungssignaldetektionseinrichtung (12,15) detektiert wird und dass diejenigen Messdaten aus den den Radon-Raum bereichsweise redundant füllenden Messdaten anhand des Herzbewegungssignals derart ausgewählt werden, dass der Radon-Raum mittels Messdaten aus möglichst bewegungsarmen Herzbewegungsphasen vollständig und homogen gefüllt ist.
